(19) Europäisches Patentamt
European Patent Office
Office européen des brevets

(11) EP 0 866 074 A1

(12) **EUROPEAN PATENT APPLICATION**
published in accordance with Art. 158(3) EPC

(43) Date of publication:
**23.09.1998 Bulletin 1998/39**

(21) Application number: **96901969.4**

(22) Date of filing: **08.02.1996**

(51) Int. Cl.$^6$: **C07K 5/12**, C12P 21/04, A61K 38/12 // (C12P21/04, C12R1:80)

(86) International application number:
**PCT/JP96/00275**

(87) International publication number:
**WO 96/25428 (22.08.1996 Gazette 1996/38)**

(84) Designated Contracting States:
**DE FR GB IT**

(30) Priority: **15.02.1995 JP 49305/95**

(71) Applicant:
**NIPPON KAYAKU KABUSHIKI KAISHA**
**Tokyo 102 (JP)**

(72) Inventors:
- **MORINO, Tomio**
**Saitama 330 (JP)**
- **MASUDA, Akira**
**Saitama 338 (JP)**
- **NISHIMOTO, Masakazu**
**Saitama 364 (JP)**

(74) Representative:
**Türk, Gille, Hrabal, Leifert**
**Brucknerstrasse 20**
**40593 Düsseldorf (DE)**

(54) **NOVEL NK374186 ANALOGUES OR PHARMACOLOGICALLY ACCEPTABLE SALTS THEREOF**

(57) NK374186 analogues such as NK374186A3 of formula (I). NK374186D3 and NK374186E3 can be obtained by culturing NK374186-producing bacteria. Other NK374186 analogues are obtained by chemically derivatizing NK374186B. These analogues and pharmacologically acceptable salts thereof are useful as the active ingredients of antitumor drugs and immunoregulators.

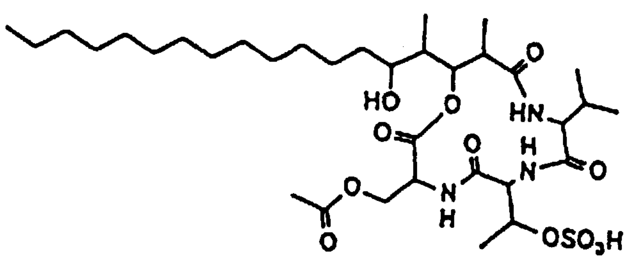

**NK374186A3** (I)

EP 0 866 074 A1

## Description

### TECHNICAL FIELD

The present invention relates to NK374186 analogues as novel biologically active substances and a method for producing the same and the use thereof.

The compounds of the present invention have an action to suppress cell proliferation and an action of modulating blastoid transformation that the compounds are promising as pharmacologically active substances to be used as anti-tumor agents, immunomodulators and the like.

### BACKGROUND ART

Conventionally known anti-tumor agents include adriamycin, bleomycin, cis-platin, and the like, while conventionally known immunomodulators include cyclosporin, mizoribin, and the like.

However, these conventional anti-tumor agents are highly toxic, unsatisfactorily. Alternatively, known immunomodulators have also insufficient effects. The discovery of a novel compound suitable for such use has been expected.

### DISCLOSURE OF INVENTION

The present inventors have intensively examined metabolic products of microorganisms. Consequently, the inventors have found that a fungal strain NK374186 described in Japanese Patent Laid-open No. 05-194571 (FERM P - 12285 deposited in the Patent Microorganism Depository, the Life Engineering and Industrial Technology Research Institute, the Agency of Industrial Science and Technology, at 1-3, Higashi 1-chome, Tsukuba-shi, Ibaraki-ken, Japan; Accession No.3870 under the Budapest Treaty) generates novel biologically active substances NK374186A3, NK374186D3 and NK374186E3. Further, the present inventors have found that novel NK374186 analogues represented by the general formula 1 according to claim 1, as well as novel substances NK374186H, NK374186I, NK374186P and NK374186PP, can be produced by chemically modifying the raw material NK374186B described in Japanese Patent Laid-open No. 05-194571.

The inventors have further found that these NK374186 analogues and pharmacologically acceptable salts thereof are useful as the effective ingredients of anti-tumor agents or immunomodulators. Thus, the present invention has bean achieved.

More specifically, the present invention relates to novel NK374186 analogues represented by the general formula 1;

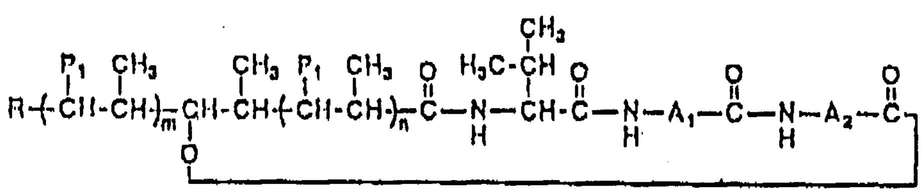

(1)

[wherein either one of m and n is 1 and the remaining one is zero; R represents a saturated alkyl group with 13 carbon atoms; $P_1$ represents hydroxyl group or phosphate group; > $A_1$ represents $>CH-CH(CH_3)-X_1$ or $>C=CH-CH_3$ wherein $X_1$ represents hydroxyl group or an inorganic acid group, or an organic acid group with one to 7 carbon atoms, the organic acid group satisfactorily containing a halogen atom; $>A_2$ represents $>CH-CH_2-X_2$ or $>C = CH_2$ wherein $X_2$ represents hydroxyl group or an inorganic acid group, or an organic acid group with one to 7 carbon atoms, the organic acid group satisfactorily containing a halogen atom, excluding a case wherein m = 0, n = 1, $P_1$ represents hydroxyl group, $-A_1$-represents $-CH[CH(CH_3)-OSO_3H]-$, and $-A_2-$ represents $- CH(CH_2OCOCH_3)-$; a case wherein m = 0, n = 1, $P_1$ represents hydroxyl group, $-A_1-$ represents $-CH[CH(CH_3)-OH]-$, and $-A_2-$ represents $-CH(CH_2OCOCH_3)-$ ; a case wherein m = 1, n = 0, $P_1$ represents hydroxyl group, $-A_1-$ represents $-CH[CH(CH_3)-OH]-$, and $-A_2$-represents $-CH(CH_2OCOCH_3)$; and a case wherein m = 1, n = 0, $P_1$ represents hydroxyl group, $-A_1-$ represents $-CH[CH(CH_3)-OH]-$, and $-A_2-$ represents $-CH(CH_2OH)]-$, or pharmacologically acceptable salts thereof.

Still further, the present invention relates to novel NK374186A3;

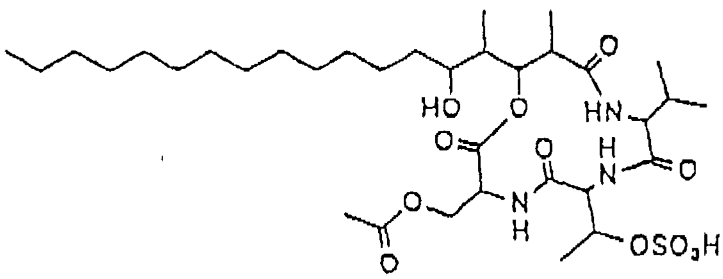

NK374186A3

novel NK374186D3;

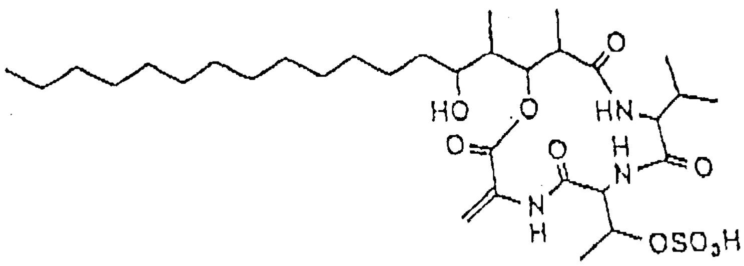

NK374186D3

or novel NK374186E3;

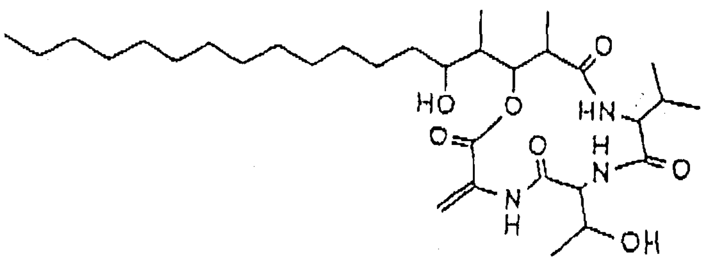

NK374186E3

or pharmaceutically acceptable salts thereof.

Still more further, the present invention relates to a method for producing biologically active substances NK374186A3, NK374186D3 and NK374186E3, comprising culturing a microorganism of genus Penicillinium and with the potency of generating NK374186A3, NK374186D3 and NK374186E3 in a culture medium to generate and accumulate biologically active substances NK374186A3, NK374186D3 and NK374186E3 in the culture and collecting these substances.

Further, the present invention relates to novel NK374186 analogues represented by the general formula 2;

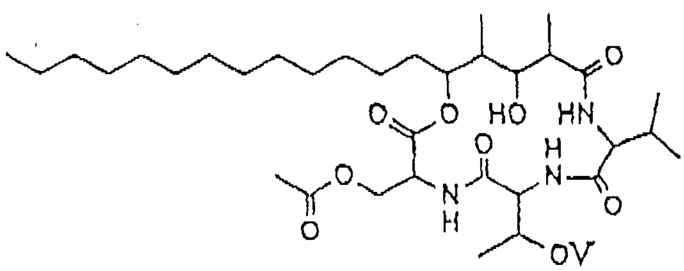

(2)

[wherein V represents an acyl group with carbon atoms of not more than 7];

or pharmacologically acceptable salts thereof.

Still yet further, the present invention relates to novel NK374186H;

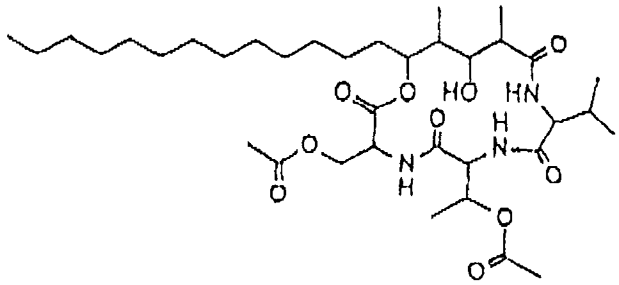

NK374186H

novel NK374186I;

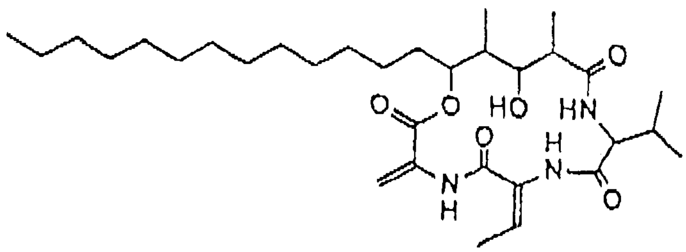

NK374186I

novel NK374186P;

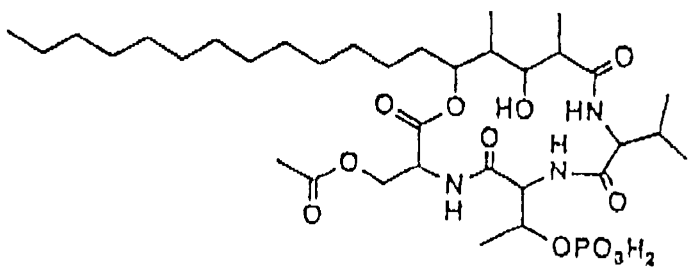

NK374186P

or novel NK374186PP;

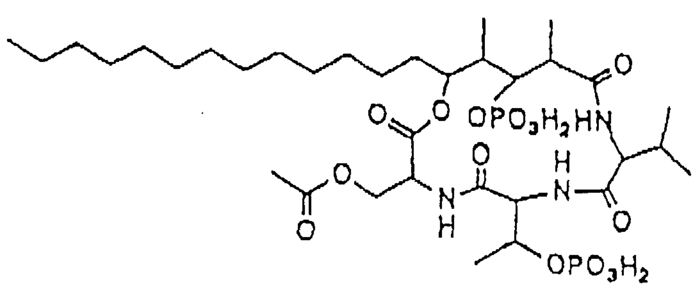

NK374186PP

or pharmacologically acceptable salts thereof.

Additionally, the present invention relates to anti-tumor agents or immunomodulators containing the novel NK374186 analogues represented by the general formula 1 or pharmacologically acceptable salts thereof as the effective ingredients; more specifically, the present invention relates to anti-tumor agents or immunomodulators containing

NK374186A3, NK374186D3, NK374186E3, NK374186H, NK374186I, NK374186P and NK374186PP or pharmacologically acceptable salts thereof as the effective ingredients.

## BRIEF DESCRIPTION OF THE DRAWINGS

Fig.1 is the ultraviolet (abbreviated as "UV" hereinbelow) absorption spectrum of NK374186A3;
Fig.2 is the H-NMR (nuclear magnetic resonance) spectrum of NK374186A3 ,as analyzed in dimethyl sulfoxide-$d_6$ ;
Fig.3 is the C-NMR spectrum of NK374186A3, as analyzed in dimethyl sulfoxide-$d_6$ ;
Fig.4 is the UV absorption spectrum of NK374186D3;
Fig.5 is the H-NMR spectrum of NK374186D3, as analyzed in dimethyl sulfoxide-$d_6$ ;
Fig.6 is the C-NMR spectrum of NK374186D3, as analyzed in dimethyl sulfoxide-$d_6$ ;
Fig.7 is the UV absorption spectrum of NK374186E3;
Fig.8 is the H-NMR spectrum of NK374186E3, as analyzed in dimethyl sulfoxide-$d_6$ ; and
Fig.9 is the C-NMR spectrum of NK374186E3, as analyzed in dimethyl sulfoxide-$d_6$ ;

## BEST MODE FOR CARRYING OUT THE INVENTION

In accordance with the present invention, the NK374186 analogues may or may not be pharmacologically acceptable salts thereof, including for example sodium salts, potassium salts, calcium salts or ammonium salts thereof.

In accordance with the present invention, the term "inorganic acid group" means a group produced by eliminating one or more hydrogens from the acid group of an inorganic acid, including for example sulfate group, phosphate group, and pyrophosphate group. In accordance with the present invention, sulfate group and phosphate group are preferable.

The term "organic acid group" means a group produced by eliminating one or more hydrogens from the acid group of an organic acid, including for example an acyl group with one to 7 carbon atoms, which may or may not have a substituent. More specifically, the acyl group includes for example a linear or branched alkylcarbonyl group, substituted or unsubstituted, such as acetyl group, propionyl group, butanoyl group and isopentanoyl group and a substituted or unsubstituted arylcarbonyl group such as benzoyl group and p-methoxybenzoyl group. Preference is given to acetyl group and propionyl group, in particular. Preferably, the halogen substituent may he fluoride atom or chloride atom, in particular.

So as to produce NK374186A3, NK374186D3 and NK374186E3 in accordance with the present invention, firstly, the aforementioned fungal strain NK374186 is aerobically cultured in a culture medium containing nutrients which the fungus can utilize, As such nutrient source, known nutrient sources conventionally used for fungal cultivation can be used, including for example glucose, fructose, glycerin, sucrose, dextrin, galactose and an organic acid, singly or in combination, as the carbon source. As the inorganic or organic nitrogen source, use may be made of ammonium chloride, ammoniumsulfate, urea, ammonium nitrate, sodium nitrate, peptone, meat extract, yeast extract, dry yeast, corn steep liquor, soy bean powder, cotton seed oil bran, casaminoic acid, Bacto-peptone, soluble vegetable protein, oat meal and the like, singly or in combination.

If necessary, furthermore, inorganic salts such as sodium chloride, calcium carbonate, magnesium sulfate, copper sulfate, iron sulfate, zinc sulfate, manganese chloride, phosphate salts and the like, can be added; additionally, organic substances such as amino acids, vitamins, and nucleic acids as well as inorganic substances can be added at appropriate amounts.

As the culturing method, liquid culture, particularly submerged agitation culture, is the most suitable. The culturing temperature is 15 °C to 35 °C; and the pH is in a neutral range or in a range of mild acidity.

By liquid culture, generally, NK374186A3, D3 and E3 are generated and accumulated during cultivation for 3 to 5 days. Cultivation is terminated when the amounts thereof in mycelia reach maximum levels. Then, the cultured broth is filtrated to separate the mycelia, to purify and isolate the objective substances.

So as to isolate and purify the inventive substances from the cultured broth, generally, use is made of any separation and purification method for use in isolating microbial metabolic products from the culture.

In other words, the cultured broth is separated from mycelia by routine filtration methods, After extracting the mycelia in methanol, an equal volume of water is added to the resulting extract solution, followed by adsorption of the substances in the resulting solution onto an adsorption resin Diaion HP-20. After washing the adsorption resin, the substances are eluted with 60 % acetone, to recover fraction 1. Subsequently, the substances are eluted with 80 % acetone to recover fraction 2. By the same manner as in the following examples, the fraction 1 is sequentially applied to Diaion HP-20SS and chromatography on a column packed with silica gel, to collect the active fractions therein, followed by concentration under reduced pressure, whereby colorless NK374186A3 and NK374183D3 can be recovered. By the same manner as in the following examples, the fraction 2 is sequentially applied to silica gel column chromatography and Sephadex LH-20 to collect the active fractions therein, followed by concentration under reduced pressure, whereby colorless NK374186E3 can be recovered.

So as to produce novel NK374186 analogues and NK374186H represented by the general formula 2 [in the formula, V represents an acyl group with not more than 7 carbon atoms] according to claim 4, in accordance with the present invention, these compounds can be synthesized from for example known NK374186B (as described in Japanese Patent Laid-open No. 05-194571), following the reaction formula 1;

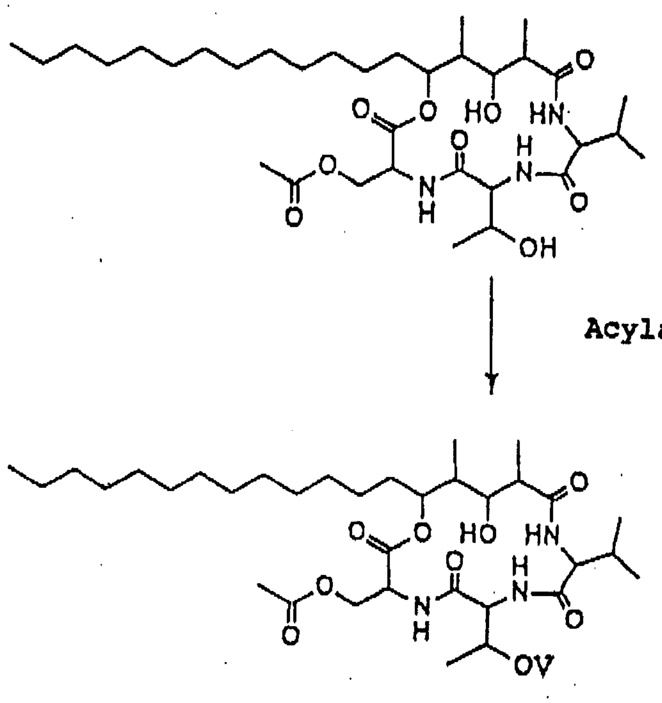

NK374186B

Acylation

(2)

Reaction formula 1.

By the acylation of NK374186B, the compounds represented by the general formula 2 can be yielded. The acyl group with not more than 7 carbon atoms, which may or may not have a substituent represented as V, includes for example a linear or branched alkylcarbonyl group, substituted or unsubstituted, such as acetyl group, propionyl group, butanoyl group and isopentanoyl group; and a substituted or unsubstituted arylcarbonyl group such as benzoyl group and p-methoxybenzoyl group. Practically, acetyl group and propionyl group are particularly preferable. The compounds represented by the general formula 2 wherein V is acyl group are produced from NK374186B by an acylating process comprising treating the NK374186B with an acylating agent or with a combination of an acylating agent and a base. The acylating agent includes for example acid halides such as acetyl chloride and benzoyl chloride; acid anhydrides such as acetic anhydride and benzoic anhydride; and a combination of carboxylic acids such as acetic acid and benzoic acid and condensation agents such as dicyclohexylcarbodiimide. The base includes for example organic bases such as pyridine, triethylamine, imidazole, tetrazole, N-methylmorpholine and 4-dimethylaminopyridine; and inorganic bases such as sodium hydrogen carbonate, potassium carbonate and sodium hydroxide. Preferably, the acylating agent is used at about 1 equivalent to 10 equivalents, while the base is used at about zero equivalent to an excess amount (the amount of the solvent).

Specific examples of the compounds represented by the general formula 2 are shown below.

NK374186H

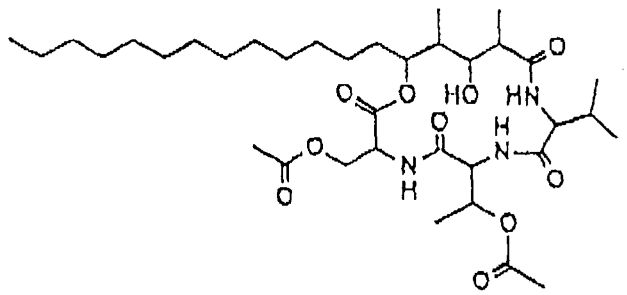

NK374186H

NK374186J

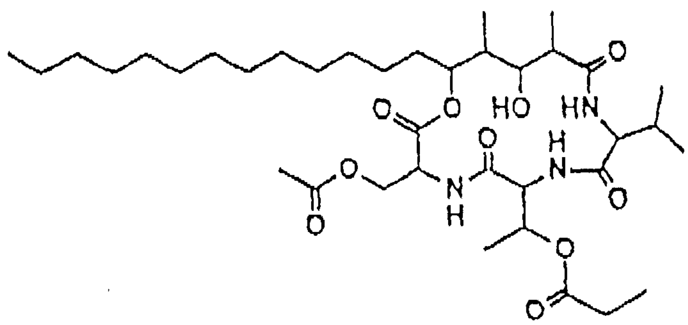

NK374186J

NK374186K

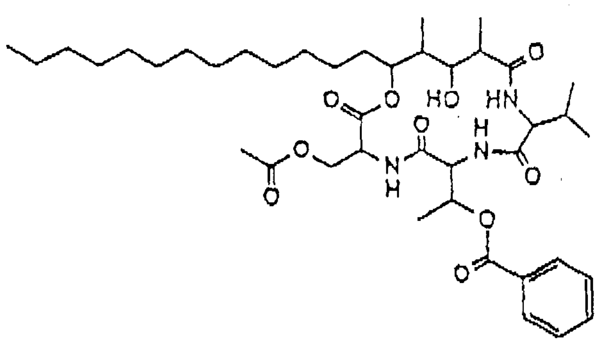

NK374186K

In accordance with the present invention, the novel NK374186I can be produced from for example known NK374186B (as described Japanese Patent Laid-open No. 05-194571), following the reaction formula 2.

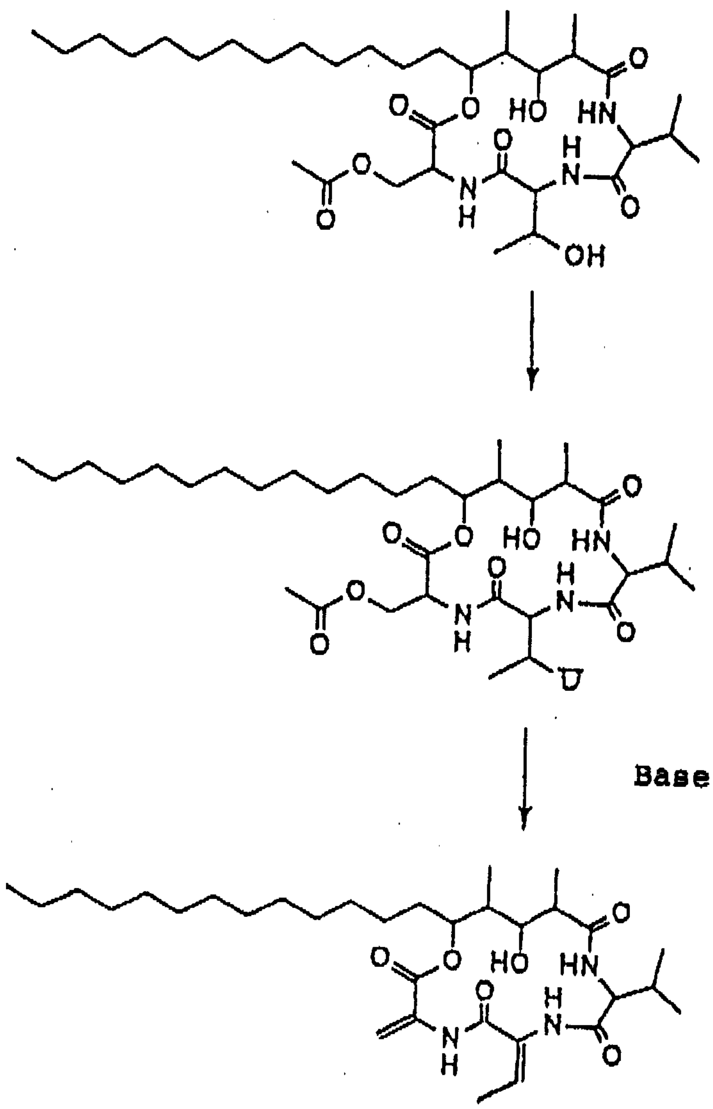


NK374186B

(b)

NK374186I

Reaction formula 2

[wherein U represents a group to be eliminated.]

In the reaction formula 2, the group to be eliminated as represented as U, includes for example sulfonyloxy groups such as methanesulfonyloxy group, toluenesulfonyloxy group, and trifluoromethanesulfonyloxy group; halogeno groups such as chloro and bromo; and acyloxy groups such as acetoxy group, propionyloxy group and benzoyloxy group. Practically, preference is given to methanesulfonyloxy group and toluenesulfonyloxy group, in particular.

The compound represented by the general formula b [wherein U represents a group to be eliminated] in the reaction formula 2 can be produced by reacting NK374186B with for example an acid chloride or an acid anhydride in the presence of a base. By treating NK374186B with for example an acid and a condensation agent, the compound represented by the general formula b can be produced. By subjecting the compound represented by the general formula b to an elimination reaction by treatment for example with a base, NK374186I can be produced. The base for producing the compound represented by the general formula b from NK374186B includes for example organic bases such as pyridine, triethylamine, imidazole, tetrazole, N-methyl morpholine and 4-dimethylaminopyridine; and inorganic bases such as sodium carbonate, sodium hydrogen carbonate and sodium hydroxide. The acid chloride includes for example sulfonylchlorides such as methanesulfonyl chloride, toluenesulfonyl chloride, and trifluoromethanesulfonyl chloride; acyl

chlorides such as acetyl chloride, propionyl chloride and benzoyl chloride; phosphorus halides and sulfur halides, such as phosphorus oxychloride, thionyl chloride and thionyl bromide.

The acid anhydride includes for example acetic anhydride, benzoic anhydride, and trifluoromethanesulfonic anhydride. The acid for the dehydration and condensation reaction includes for example carboxylic acids such as acetic acid and benzoic acid; sulfonic acids such as methanesulfonic acid and toluenesulfonic acid; and mineral acids such as hydrochloric acid and hydrobromic acid. The condensation agent includes for example dicyclohexylcarbodiimide, diethyl azodicarboxylic acid and carbonyldiimidazole. Preferably, the acid chloride or acid anhydride is used at about 1 to 10 equivalents, while the base is used at about zero equivalent to an excess amount (the amount of the solvent). For the dehydration and condensation reaction, the acid is satisfactorily used at about 1 to 10 equivalents, while the dehydration and condensation agent is used at about 1 equivalent to 10 equivalents.

The elimination reaction for producing NK374186I from the compound represented by the general formula b [wherein U represents a group to be eliminated] following the reaction formula 2 comprises treating the compound with inorganic bases such as lithium hydroxide, sodium hydroxide, and potassium carbonate, organic bases such as sodium methoxide, potassium t-butoxide, triethylamine, 1,8-diazabicyclo[5.4.0]undecene, and 4-dimethylaminopyridine, organic metals such as butyl lithium, 4-butylmagnesium chloride in a solvent for example methylene chloride, tetrahydrofuran, methanol and water.

Specific examples of the compound represented by the general formula b in the reaction formula 2 are shown in the following structural formulas.

Compound b1

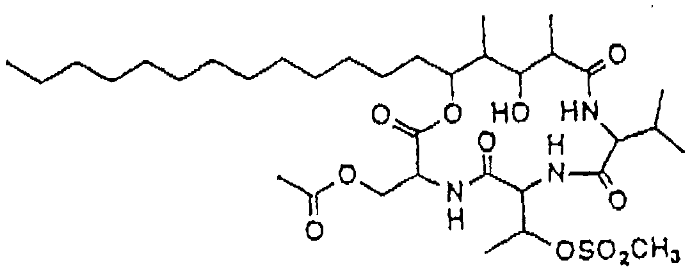

(b 1)

Compound b2

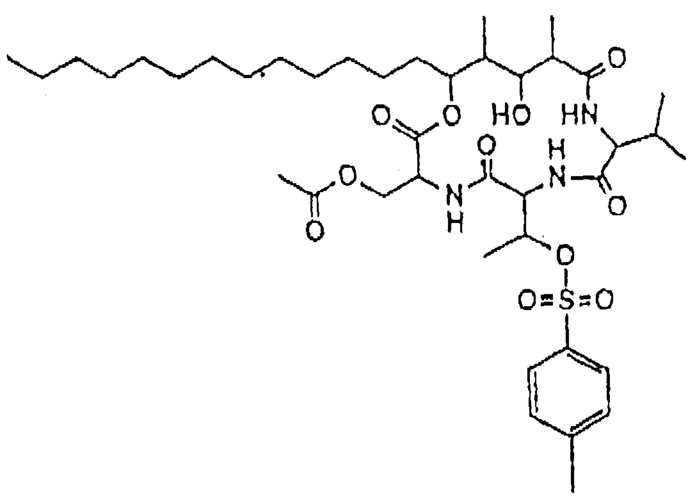

(b 2)

Compound b3

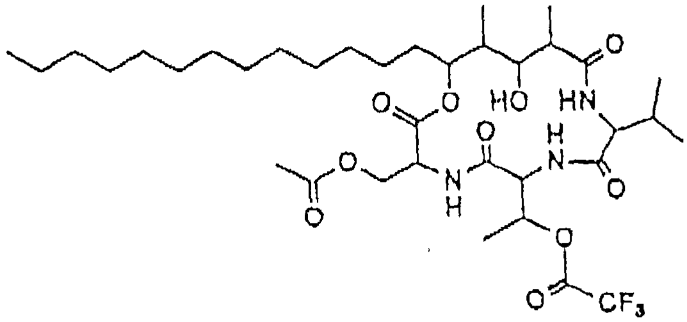

(b 3)

In accordance with the present invention, furthermore, NK374186P and NK374186PP are produced from for example known NK374186B (as described in Japanese Patent Laid-open No. 05-194571), following the reaction formula 3.

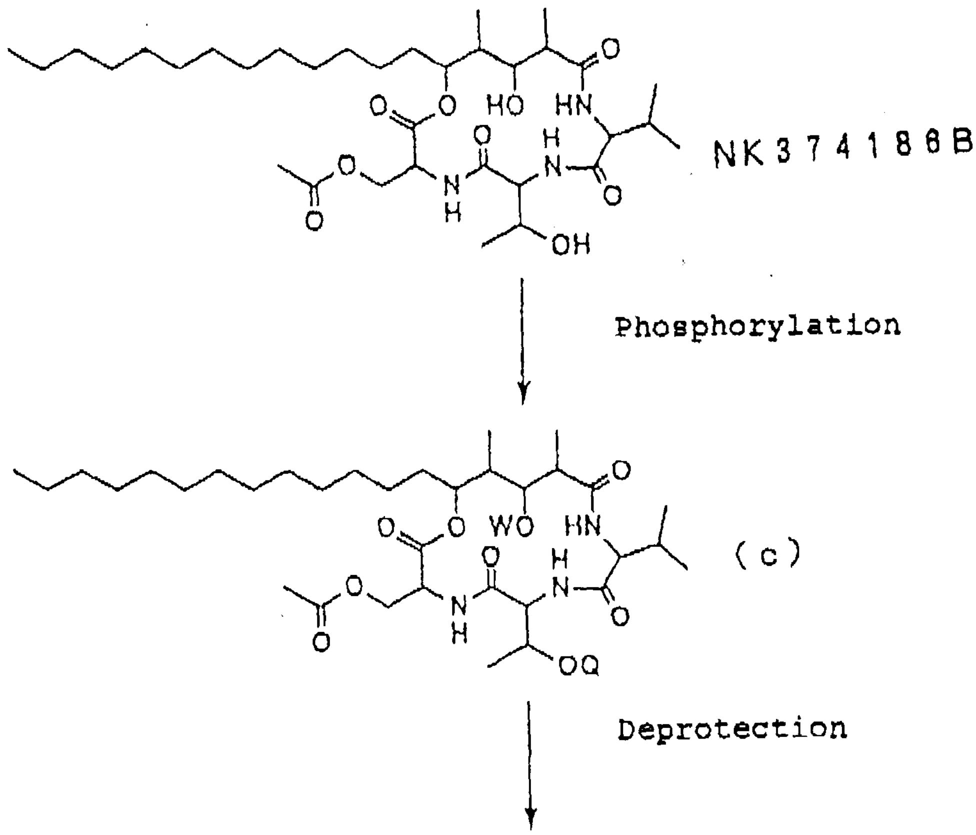

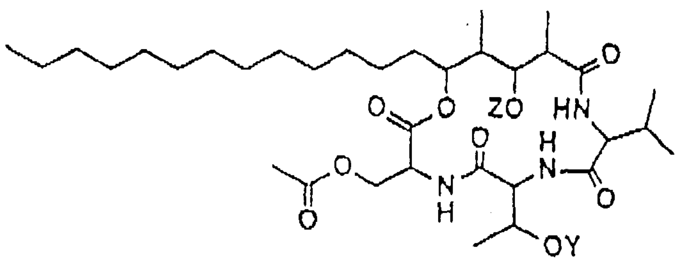

(3)

Reaction formula 3

Reaction formula 3

[In the formula, Y represents phospho group; Z represents hydrogen atom or phospho group; Q represents a phospho group protected by a protective group; and W represents hydrogen atom or a phospho group protected by a protective group].

In the reaction formula 3, the protective group of the phospho groups in Q and W includes for example a substituted or unsubstituted alkyl group, or a substituted or unsubstituted aryl group. The alkyl group includes for example methyl, 2-cyanoethyl, benzyl, and o-xylyl. The aryl group includes for example phenyl and p-methoxyphenyl.

More specifically, NK374186B reacts with for example a phosphorylating agent in the presence of a base for phosphorylation, to produce the compound represented by the general formula c [wherein Q represents a phospho group protected by a protective group; W represents hydrogen atom or a phospho group protected by a protective group] in the reaction formula 3. Also, NK374186B reacts with for example a phosphitizing agent in the presence of a base, followed by treatment with an oxidizing agent, to yield the compound represented by the general formula c. When the compound represented by the general formula c is subjected to for example contact reduction, the compound represented by the general formula 3 in the reaction formula 3 can be yielded.

The base for the phosphatization for producing the compound represented by the general formula c in the reaction formula 3 from NK374186B includes for example organic bases such as pyridine, triethylamine, imidazole, tetrazole, N-methyl morpholine, and 4-dimethylaminopyridine; and organic metals such as butyl lithium and 4-butylmagnesium chloride. The phosphorylating agent includes for example dibenzyl phosphorochloridate, and diphenyl phosphorochloridate. The phosphitizing agent includes for example dibenzyl diethyl phosphoroamidite, and N,N-diethyl-1,5-dihydro-2,4,3-benzodioxaphosphepine-3-amine. The oxidizing agent includes for example 3-chloroperbenzoic acid and iodine. Preferably, the phosphorylating agent or phosphitizing agent is used at about 1 to 10 equivalents, while the base is used at zero equivalent to an excess amount (the amount of the solvent).

The contact reduction for producing the compound represented by the general formula 3 [wherein Y represents phospho group; and Z represents hydrogen atom or phospho group] from the compound represented by the general formula c in the reaction formula 3 comprises agitation in hydrogen atmosphere or under purging of hydrogen gas in the presence of a contact reduction catalyst such as 10 % palladium - carbon and 20 % palladium hydroxide - carbon in a solvent such as ethanol and acetic acid.

Specific examples of the compound represented by the general formula c in the reaction formula 3 are shown in the following structural formulas.

Compound c1

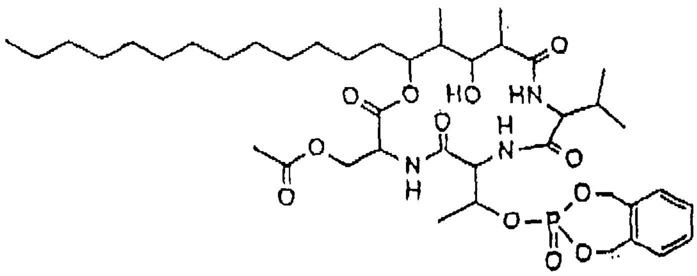

(c 1)

Compound c2

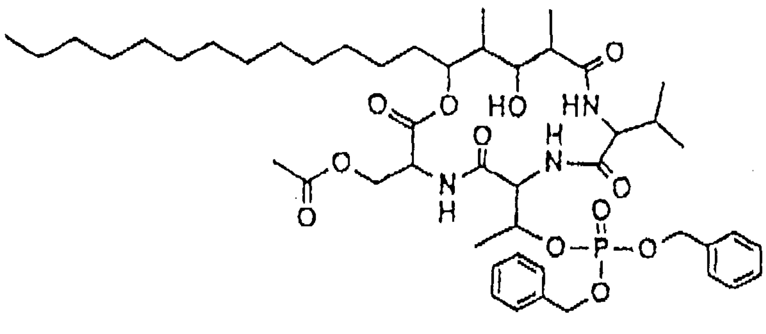

(c 2)

Compound c3

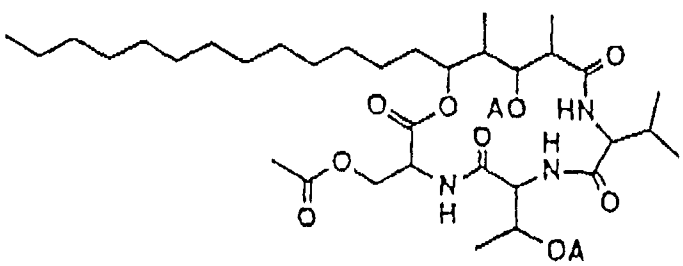

(c 3)

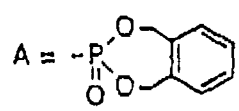

Specific examples of the compound represented by the general formula 3 in the reaction formula 3 include NK374186P and NK374186PP, as shown in the aforementioned structural formulas.

The novel NK374186 analogues represented by the general formula 1 according to claim 1, namely NK374186A3, NK374186D3, NK374186E3, NK374186H, NK374186I, NK374186P and NK374186PP (referred to as "NK374186" hereinbelow), are promising as pharmaceutical products such as anti-cancer agents and immunomodulators, as described below.

Any of various methods conventionally used for formulation and administration is applicable when the NK374186 is intended for use as pharmaceutical products. In other words, injection, oral administration, intra-rectal administration and the like are satisfactory as the administration method. As to the formulation, formulations such as injections, powders, tablets, and suppositories are applicable. For the formulation, various co-agents, namely carriers and other auxiliary agents, for example stabilisers, antiseptics, soothing agents, and emulsifiers, may be used if necessary, unless these substances adversely affect the formulation.

The content of the NK374186 can be modified in a wide range, depending on the formulation; generally, the formulation of the NK374186 contains 0.01 to 100 % by weight (abbreviated as "wt %" hereinbelow), preferably 0.1 to 70 wt % of the NK374186, with the remaining part being occupied by carriers and other auxiliary agents for general use for pharmaceutical agents.

The dose of the NK3741863 varies, depending on the symptom of a patient, but generally, the dose is about 0.01 to 800 mg per day per adult. For continuous dosing, if necessary, the dose per day should be reduced.

Experimental Examples

1. Activity on the suppression of cancer cell proliferation

Cells were cultured in an RPMI culture medium supplemented with 10 % calf fetus serum in the presence of 5 % $CO_2$ at 37 °C. The cells were inoculated and cultured in a 96-well plate for one day, followed by addition of one of the pharmaceutical compounds. The cells were treated with the compounds for 3 days, and the effects were assessed by the proliferation-suppressed MTT method. The activities of the compounds on the suppression of the proliferation of Cancer cells are shown below. The cancer cells were of a cell line A2780 derived from human ovarian cancer.

Table 1

| Activities of NK374186 on the suppression of cancer cell proliferation | |
|---|---|
| Compound | IC50(μg/ml) |
| A3 | 26 |
| D3 | 20 |
| E3 | 3.7 |
| H | 12 |
| I | 9.7 |
| P | 33 |
| PP | >50 |

Table 1 apparently shows that NK374186A3, D3, E3, H, I and P have activities on the suppression of the proliferation of the cancer cells.

2. Immunomodulating activity during blastoid transformation

For cultivation, RPMI 1640 medium supplemented with 10 % calf fetus serum, 25 mM HEPS buffer, 100 μg/ml streptomycin and 100 units/ml streptomycin and 100 units/ml penicillin G in a 96-well flat-bottom microplate (coaster) was used. As a mitogen, 2 ng/ml OKT-3 antibody was used.

Human peripheral T cells Were collected from blood drawn with heparin by the density-gradient centrifugation method. 200,000 cells and test compounds at individual concentrations were added to each well to a total volume of 0.2 ml, for 72-hr cultivation. Eight hours before termination of the cultivation, 37 kBq $^3H$ - thymidine was added to determine the incorporation thereof into the cells. The effects were evaluated on the basis of the ratio of the incorporated $^3H$ - thymidine in the groups with addition of the test compounds to the incorporated $^3H$ - thymidine in the control group (Japanese Immunology Association, Methods for Experiment Immunology, pp. 2267 - 2276). The results are shown in Table 2.

Table 2

| NK374186 action on human blastoid transformation via OKT-3 | | | | | | | |
|---|---|---|---|---|---|---|---|
| Concentration | A3 | D3 | E3 | H | I | P | PP |
| 0.03 | 103.1 | 112.9 | 99.4 | 105.1 | 108.7 | 99.6 | 99.2 |
| 0.1 | 97.6 | 99.1 | 106.1 | 100.9 | 93.3 | 100.6 | 99.9 |
| 0.3 | 103.5 | 98.7 | 78.8 | 104.2 | 87.5 | 102.6 | 98.3 |
| 1.0 | 121.0 | 111.0 | 78.8 | 91.6 | 69.8 | 119.7 | 109.7 |
| 3.0 | 129.1 | 119.9 | 7.9 | 67.0 | 27.1 | 131.4 | 109.3 |
| 10.0 | 88.1 | 82.5 | 0.2 | 0.2 | 0.2 | 97.7 | 87.5 |
| 30.0 | 60.6 | 67.4 | 0.2 | 0.2 | 0.1 | 69.3 | 81.3 |

As apparently shown in Table 2, the NK374186 of the present invention promotes the blastoid transformation via OKT-3 at lower concentrations, but suppresses the transformation at higher concentrations.

3. Immunomodulation of NK374186 on graft vs host reaction (abbreviated as "GVH" below)

The spleen weight method by Fords, et al. [W.L. Ford et al., Handbook of Experimental Immunology 30, D.M. Weir, ed., Blackwell Scientific Publications, 1978, pp. 1] was followed. Spleen cells of $5 \times 10^6$, separated from a C57BL/6 mouse (8 weeks of age, male) as a donor, were transplanted peritoneally in a BDF mouse (7 weeks of age, male) as a recipient. Simultaneously, NK374186, a control agent or a solvent control (0.25 % gum arabic) was injected subcutaneously in the dorsal skin of the BDF mouse. The NK374186 and the control agent were preliminarily dissolved in 0.25 % gum arabic. Furthermore, on day 1 and day 2, the same administration was conducted, and 7 days after the transplantation, the spleen was weighed. The results are shown in Table 3.

Table 3

| Effects of NK374186 on GVH | | | |
|---|---|---|---|
| Sample | Dose (mg/kg) | Spleen weight/body weight [$X10^{-3}$] | Index |
| Untreated | | 4.83 | 1.00 |
| Treated | | 8.26 | 1.71 |
| 0.25 % gum arabic | | 7.00 | 1.45 |
| NK374186A3 | 0.5 | 9.35 | 1.94 |
| NK374186A3 | 5.0 | 11.35 | 2.34 |
| NK374186A3 | 50.0 | 12.01 | 2.49 |
| NK374186P | 0.5 | 10.01 | 2.07 |
| NK374186P | 5.0 | 11.45 | 2.37 |
| NK374186P | 50.0 | 12.00 | 2.48 |

As apparently shown above, depending on the dose, the ratio of the spleen weight to the body weight increases in the NK374186A3 and NK374186P groups, which indicates significant GVH suppression.

The present invention will be described in the following examples is a way of illustration, and is not for limitation. Various modifications are possible.

The compounds of the present invention are specifically described in examples.

Example 1

NK374186A3, D3 and E3 are generated during cultivation.

1. Fermentation

A seed culture medium of the following composition (100 ml) was placed in a 500-ml Erlenmeyer flask, and sterilized in an autoclave at 120 °C for 20 minutes. One platinum loop of the NK374186 strain (FERM-BP 12285) was inoculated in the medium for cultivation under conditions of 27 °C and 200 rpm for 2 days. The resulting culture was defined as a first seed culture.

| Composition of seed culture | (%) |
|---|---|
| Glucose | 2.0 |
| Sucrose | 1.0 |
| Lactose | 1.0 |
| Glycerin | 0.2 |
| Soy bean powder | 2.0 |
| Polypeptone | 0.5 |
| Sodium nitrate | 0.2 |
| Magnesium sulfate | 0.1 |
| Silicone | 0.05 |
| TOHO NO.1 | 0.03 |
| Tap water | |

The seed culture was then cultured as follows. Twenty liters of the culture medium of the same composition as the medium for the first culture were placed in a 30-liter jar fermentor, and was then sterilized. The first seed culture of 200 ml was aseptically transferred and cultured therein at 25 °C, under purging of air at 20 l/min and agitation at 250 rpm for three days. The resulting culture was defined as a second seed culture.

The main cultivation was carried out as follows. Into a 200-liter tank was placed a production medium of 150 liters and of the same composition as the seed culture medium except that the glycerin concentration was modified to 1.0 %, followed by sterilization. The second seed culture of 2 liters, as recovered by the method described above, was aseptically transferred in the tank, which was then cultured at 25 °C therein under air purging of 150 liters/min and agitation at 250 rpm for 4 days. The cultured broth recovered from four tanks of a 200-liter volume was filtered by means of a filter press to separate a filtrate and the mycelia.

2. Purification

Into the resulting mycelia was added methanol (200 liters), for extraction under agitation for 3 hours. The extract solution was separated through filtration under suction. An equal volume of water was added to the resulting extract solution. The solution was passed through a 20-liter HP-20 column, followed by washing in 50 % methanol and subsequent elution with 60 % acetone (20 liters), to yield fraction 1. Then, the extract was eluted with 80 % acetone (30 liters) to yield fraction 2.

After concentration and drying up the fraction 1, the dried product was dissolved in 0.2 liter of water, which was then passed through a 2-liter HP-20ss column and eluted with acetone of a 50 % - 70 % concentration gradient. Fraction 1A and fraction 1B were sequentially recovered during elution. The fractions 1A and 1B were individually applied to an LH-20 column equilibrated with a solution of chloroform and methanol (1 : 1) to recover NK374186A3 (800 mg) from the fraction 1A and recover NK374186D3 (130 mg) from the fraction 1B.

After concentration and drying, the fraction 2 was applied to a 5-liter silica gel column, followed by washing in chloroform (5 liters). Subsequently, the column was eluted with a solution of chloroform and methanol (50 : 1), to recover

fraction 2A. The fraction 2A was sequentially applied to a silica gel column and an LH-20 column to be then eluted with a solution of hexane and acetone (3 : 1), to recover NK374186E3 (500 mg).

The resulting physico-chemical properties of the individual ingredients are shown below.

NM374186A3

MW = 735 (FAB - Negative Spectrum)

Ultraviolet absorption spectrum is shown in Fig.1.

H-NMR spectrum in dimethyl sulfoxide-$d_6$ is shown in Fig. 2.
C-NMR spectrum in dimethyl sulfoxide-$d_6$ is shown in Fig.3.

NK374186D3

MW = 675 (FAB - Negative Spectrum)

Ultraviolet absorption spectra are shown in Fig.4.

H-NMR spectrum in dimethyl sulfoxide-$d_6$ is shown in Fig.5.
C-NMR spectrum in dimethyl sulfoxide-$d_6$ is shown in Fig.6.

NK374186E3

MW = 595 (FAB - Negative Spectrum)

Ultraviolet absorption spectrum is shown in Fig.7.

H-NMR spectrum in dimethyl sulfoxide-$d_6$ is shown in Fig.8.
C-NMR spectrum in dimethyl sulfoxide-$d_6$ is shown in Fig.9.

Example 2: Production of NK374186H

NK374186B (42.6 mg, 0.065 mmol) was dissolved in pyridine (6.5 ml), followed by addition of acetic anhydride (30.7 μ 1, 0.325 mmol) for overnight agitation at ambient temperature. The reaction solution was concentrated under reduced pressure, and the resulting residue was purified by silica gel chromatography (5 g of silica gel; eluted with 2 % methanol - chloroform), to recover NK374186H (36.0 mg, 79 %).

[NK374186H]

$^1$H-NMR(200MHz, DMSO-$d_6$)δ:8.31(1H, d), 8.18(1H, d), 7.95(1H, d), 5.42(1H,d), 5.22(1H,m), 4.90(1H,m), 4.67(1H,m), 4.54(2H, m), 3.98(2H,m), 3.62(1H,m), 2.22(1H,m), 2.07(1H,m), 2.00(3H, s), 1.95(3H,s), 1.71(1H,m), 1.50(2H,m), 1.23(22H,m), 1.12(6H, m), 0.89(9H, m), 0.76(3H, d);
FAB-MS m/z:698[$(M+H)^+$].

Example 3: Production of compound b1

NK374186B (52.7 mg, 0.080 mmol) and 4-dimethylaminopyridine (10.1 mg, 0.083 mmol) were dissolved in methylene chloride (0.7 ml), followed by addition of triethylamine (15.0 μ l, 0.108 mmol) and methanesulfonyl chloride (7.4 μl, 0.096 mmol) at -40 °C in argon atmosphere. The resulting mixture was agitated under ice cooling for 1.5 hours, followed by further addition of triethylamine (15.0 μ l, 0.108 mmol) and methanesulfonyl chloride (7.4 μl, 0.096 mmol) at -40 °C in argon atmosphere and subsequent overnight agitation at 5 °C. To the reaction solution was added an aqueous saturated sodium hydrogen carbonate solution, which was then extracted in methylene chloride. The resulting organic phase was washed in saturated sodium chloride solution and dried over anhydrous magnesium sulfate, prior to distilling off the solvent under reduced pressure. The residue was purified by silica gel chromatography (silica gel of 8 g; eluted with 0 - 2 % methanol - chloroform) to recover the compound b1 (45.6 mg; 78 %).

[Compound b1]

FAB-MS M/Z:734 [(M + H)$^+$]

Example 4: Production of NK374186I

Compound b1 (45.0 mg, 0.061 mmol) was dissolved in tetrahydrofuran (0.7 ml), followed by addition of 1,8-diazabicyclo[5.4.0]undecene (10.1 μ l, 0.067 mmol) under agitation. Furthermore, 1,8-azabicyclo[5.4.0]undecene (5.1 μl, 0.034 mmol) was added 1.5 hours later, followed by agitation for 4 hours. Ethyl acetate was added to the reaction solution, and the resulting solution was washed sequentially in an aqueous 1N hydrochloric acid solution and in water, and further washed in saturated sodium chloride solution, and dried over anhydrous magnesium sulfate. The solvent was distilled off under reduced pressure, and the resulting residue was purified by silica gel chromatography (silica gel of 3 g, eluted with 25 - 35 % ethyl acetate - hexane), to recover NK374186I (21.4 mg, 61 %).

[NK374186I]

$^1$H-NMR(200MHz, $CDCl_3$)δ:8.15(1H, brs), 7.77(1H, brs), 6.97(1H, q), 6.63(1H, s), 6.35(1H, m), 5.98(1H, s), 4.91(1H, brs), 4.28(1H, m), 3.83(1H, m), 3.42(1H, dd), 2.28(2H, m), 1.87(1H, m), 1.80(3H,d), 1.40(2H,m), 1.24(22H,m), 0.96(6H,m), 0.87(6H, m);
FAB-MS m/z:578[(M+H)$^+$].

Example 5: Production of compound c1

NK374186B (61.2 mg, 0.093 mmol) and 1H-tetrazole (32.6 mg, 0.465 mmol) were dissolved in methylene chloride (2 ml), followed by addition of N,N-dimethyl-1,5 -dihydro-2,4,3-benzodioxaphosphepine-3-amine (24.1 μl, 0.112 mmol) in argon atmosphere and subsequent agitation at ambient temperature. Thirty minutes later, furthermore, N,N-dimethyl-1,5 - dihydro-2,4,3-benzodioxaphosphepine-3-amine (12.0 μl, 0.056 mmol) was added, for subsequent agitation for 20 minutes. Water (50 μl) was added to the resulting mixture for 10-min agitation, which was then cooled to -40 °C, followed by addition of 3-chloroperbenzoic acid (140 mg, 0.811 mmol). After the temperature of the resulting mixture resumed ambient temperature, the mixture was agitated for another 10 minutes. To the reaction solution was added an aqueous 10 % sodium sulfite solution (10 ml) for agitation, prior to extraction in ethyl acetate. The resulting organic phase was washed in an aqueous saturated sodium hydrogen carbonate solution and then in saturated sodium chloride solution and dried over anhydrous sodium sulfate, to distill off the solvent under reduced pressure. The residue was purified by silica gel chromatography (silica gel of 15 g, eluted with 1 % methanol - chloroform), to recover the compound c1 (39.4 mg, 51 %).

[Compound c1]

FAB-MS M/Z:838[(M+H)$^+$], 860[(M+Na)$^+$]

Example 6: Production of NK374186P

The compound c1 (29.1 mg, 0.035 mmol) was dissolved in an aqueous 80 % ethanol solution (5 ml), followed by addition of 10 % palladium - carbon (at a water content of 50 %, 10 mg) and subsequent agitation under purging of hydrogen gas for 40 minutes. The reaction solution was filtered through Celite, to remove the palladium - carbon, Into the resulting filtrate was added aqueous conc. ammonia (0.1 ml), prior to concentration under reduced pressure. The concentrate was dissolved in water (1 ml) for freeze-drying, to recover NK374186P as the ammonium salt thereof (25.9 mg, 96 %).

[NK374186P]

$^1$H-NMR(200MHz, DMSO-$d_6$)δ:8.18(1H,d), 7.94(1H,d), 7.88(1H,d), 4.90(2H, m), 4.60(2H, m), 4.35(2H, m), 3.99(2H, m), 3.59(1H, m), 2.12(2H,m), 2.00(3H,s), 1.60(3H,m), 1.24(22H,m), 1.12(6H, m), 0.87(9H, m), 0.72(1H, d);
FAB-MS m/z:758[(M+Na)$^+$], 780[(M+2Na)$^+$].

Example 7: Production of compound c3

NK374186B (65.5 mg, 0.100 mmol) and 1H-tetrazole (50.0 mg, 0.714 mmol) were dissolved in methylene chloride (2 ml), followed by addition of N,N-dimethyl-1,5 -dihydro-2,4,3-benzodioxaphosphepine-3-amine (0.104 ml, 0.482 mmol) in argon atmosphere and subsequent agitation at ambient temperature for 15 minutes. Water (0.05 ml) was added to the resulting mixture for 15-min agitation, which was then cooled to -40 °C followed by addition of 3-chloroperbenzoic acid (148 mg, 0.858 mmol). After the temperature of the resulting mixture resumed ambient temperature, the mixture was agitated for another 15 minutes. To the reaction solution was added an aqueous 10 % sodium sulfite solution (10 ml) for agitation, prior to extraction in ethyl acetate. The resulting organic phase was washed in an aqueous saturated sodium hydrogen carbonate solution and then in saturated sodium chloride solution and dried over anhydrous sodium sulfate, to distill off the solvent under reduced pressure. The residue was purified by silica gel chromatography (silica gel of 15 g, eluted with 2 % methanol - chloroform), to recover the compound c3 (93.8 mg, 93 %). Compound c3

FAB-MS m/z:1020[$(M+H)^+$],1042[$(M+Na)^+$]

Example 8: Production of NK374186PP

The compound c3 (80.0 mg, 0.079 mmol) was dissolved in an aqueous 75 % ethanol solution (20 ml), followed by addition of 10 % palladium - carbon (at a water content of 50 %, 40 mg) and subsequent agitation under purging of hydrogen gas for one hour. The reaction solution was filtered through Celite, to remove the palladium - carbon. Into the resulting filtrate was added aqueous conc. ammonia (0.6 ml), prior to concentration under reduced pressure. The concentrate was dissolved in water (1 ml) for freeze-drying, to recover NK374186PP as the ammonium salt thereof (65.1 mg, 93 %).

[NK374186PP]

$^1$H-NMR(200MHz, DMSO-$d_6$)$\delta$:9.64(1H,d), 8.65(1H,d), 7.82(1H,d), 4.70-4.00(8H, m), 2.23(3H, m), 1.93(3H, s), 1.80(1H, m), 1.50(2H, m), 1.32(6H, m), 1.24(22H, m), 1.01(3H, t), 0.83(9H, m);
FAB-MS m/z:838[$(M+Na)^+$], 860[$(M+2Na)^+$], 882[$M+3Na]^+$].

INDUSTRIAL APPLICABILITY

The NK374186 analogues and pharmacologically acceptable salts thereof in accordance with the present invention are promising as the effective ingredients of novel anti-cancer agents and novel immunomodulators.

## Claims

1. Novel NK374186 analogues represented by the general formula 1;

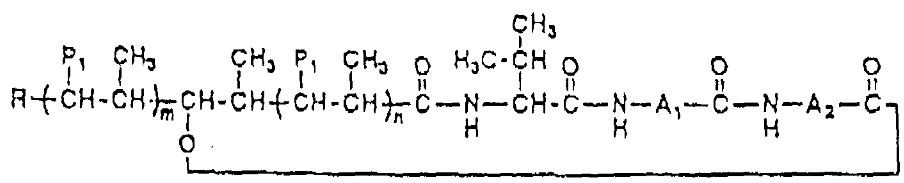
(1)

wherein either one of m and n is 1 and the remaining one is zero; R represents a saturated alkyl group with 13 carbon atoms; $P_1$ represents hydroxyl group or phosphate group; > $A_1$ represents >CH-CH($CH_3$)-$X_1$ or >C = CH-$CH_3$ wherein $X_1$ represents hydroxyl group or an inorganic acid group, or an organic acid group with one to 7 carbon atoms, the organic acid group satisfactorily containing a halogen atom; >$A_2$ represents >CH-$CH_2$-$X_2$ or >C = $CH_2$ wherein $X_2$ represents hydroxyl group or an inorganic acid group, or an organic acid group with one to 7 carbon atoms, the organic acid group satisfactorily containing a halogen atom, excluding a case wherein m = 0, n = 1, $P_1$ represents hydroxyl group, -$A_1$- represents - CH[CH($CH_3$)-$OSO_3H$]-, and -$A_2$- represents -CH($CH_2OCOCH_3$)-; a case wherein m = 0, n = 1, $P_1$ represents hydroxyl group, -$A_1$- represents -CH[CH($CH_3$)-OH]-, and -$A_2$- represents -CH($CH_2OCOCH_3$)- ; a case wherein m = 1, n = 0, $P_1$ represents hydroxyl group, -$A_1$- represents -CH[CH($CH_3$)-OH]-, and -$A_2$- represents -CH($CH_2OCOCH_3$); and a case wherein m = 1, n = 0, $P_1$ represents hydroxyl group, -$A_1$- represents -CH[CH($CH_3$)-OH]-, and -$A_2$- represents -CH($CH_2$OH)-, or pharmacologically

acceptable salts thereof.

**2.** Novel NK374186A3;

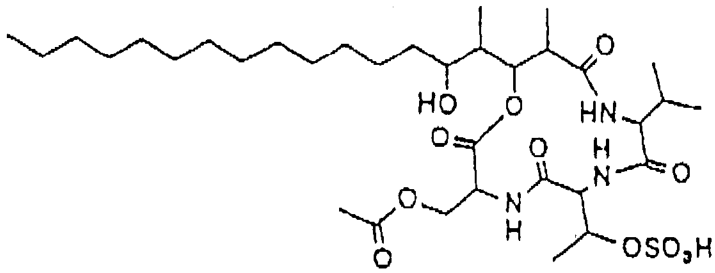

NK374186A3

novel NK374186D3;

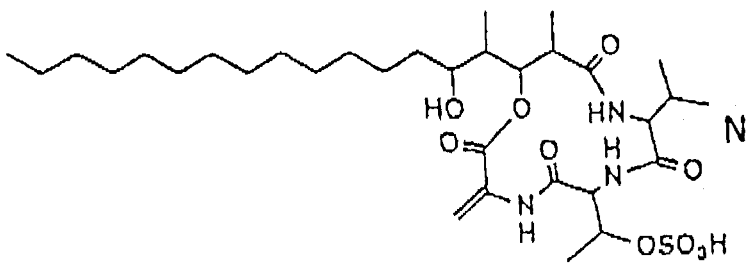

NK374186D3

or novel NK374186E3;

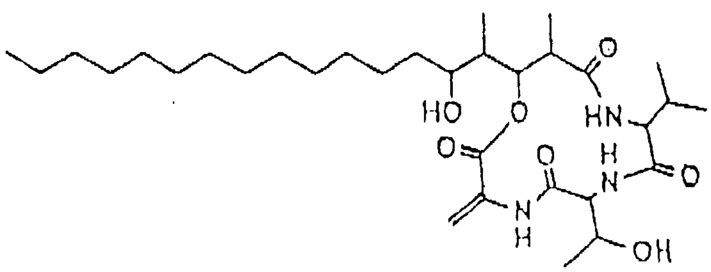

NK374186E3

as a NK374186 analogue according to claim 1, or pharmaceutically acceptable salts thereof.

**3.** A method for producing biologically active substances NK374186A3, NK374186D3 and NK374186E3, comprising culturing a microorganism of genus <u>Penicillinium</u> and with the potency of generating NK374186A3, NK374186D3 and NK374186E3 in a culture medium to generate and accumulate biologically active substances NK374186A3, NK374186D3 and NK374186E3 in the culture and collecting these substances.

**4.** Novel NK374186 analogues represented by the general formula 2;

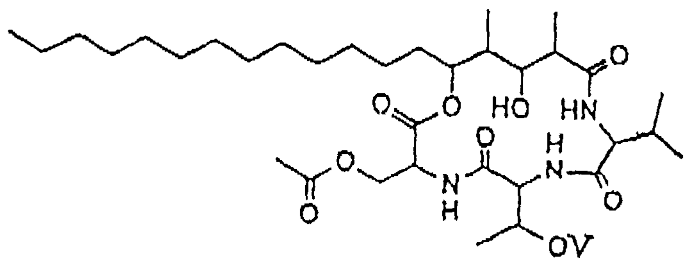

(2)

wherein V represents an acyl group with carbon atoms of not more than 7]; or pharmacologically acceptable salts thereof.

**5.** Novel NK374186H;

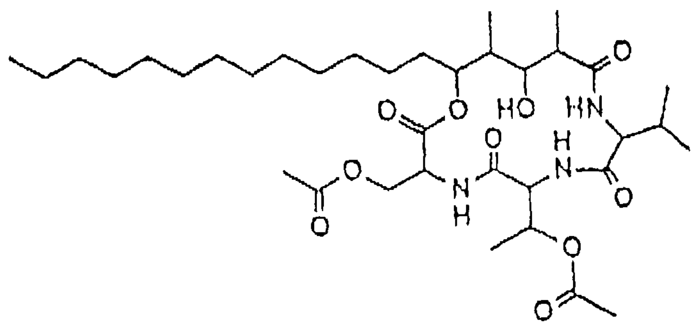

NK374186H

novel NK374186I;

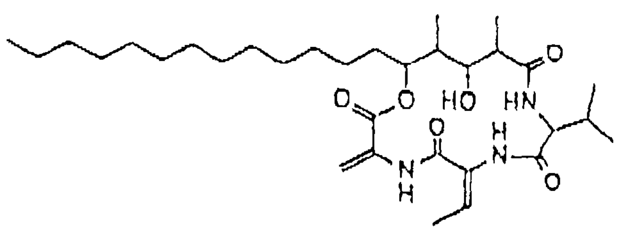

NK374186I

novel NK374186P;

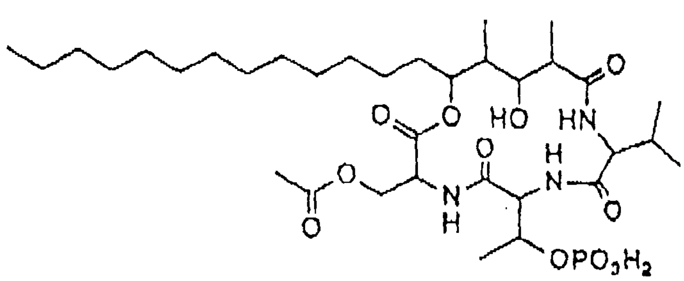

NK374186P

or novel NK374186PP;

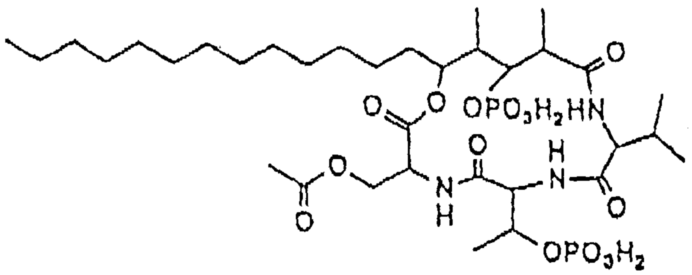

NK374186PP

as a NK374186 analogue according to claim 1, or pharmacologically acceptable salts thereof.

6. An anti-tumor agent or immunomodulator containing novel NK374186 analogues according to claim 1 or pharmacologically acceptable salts thereof as the effective ingredients and further containing a auxiliary agent for use in pharmaceutical products.

7. An anti-tumor agent or immunomodulator containing NK374186A3, NK374186D3, NK374186E3, NK374186H, NK374186I, NK374186P and NK374186PP according to claims 2 and 5, or pharmacologically acceptable salts thereof as the effective ingredients and further containing a auxiliary agent for use in pharmaceutical products.

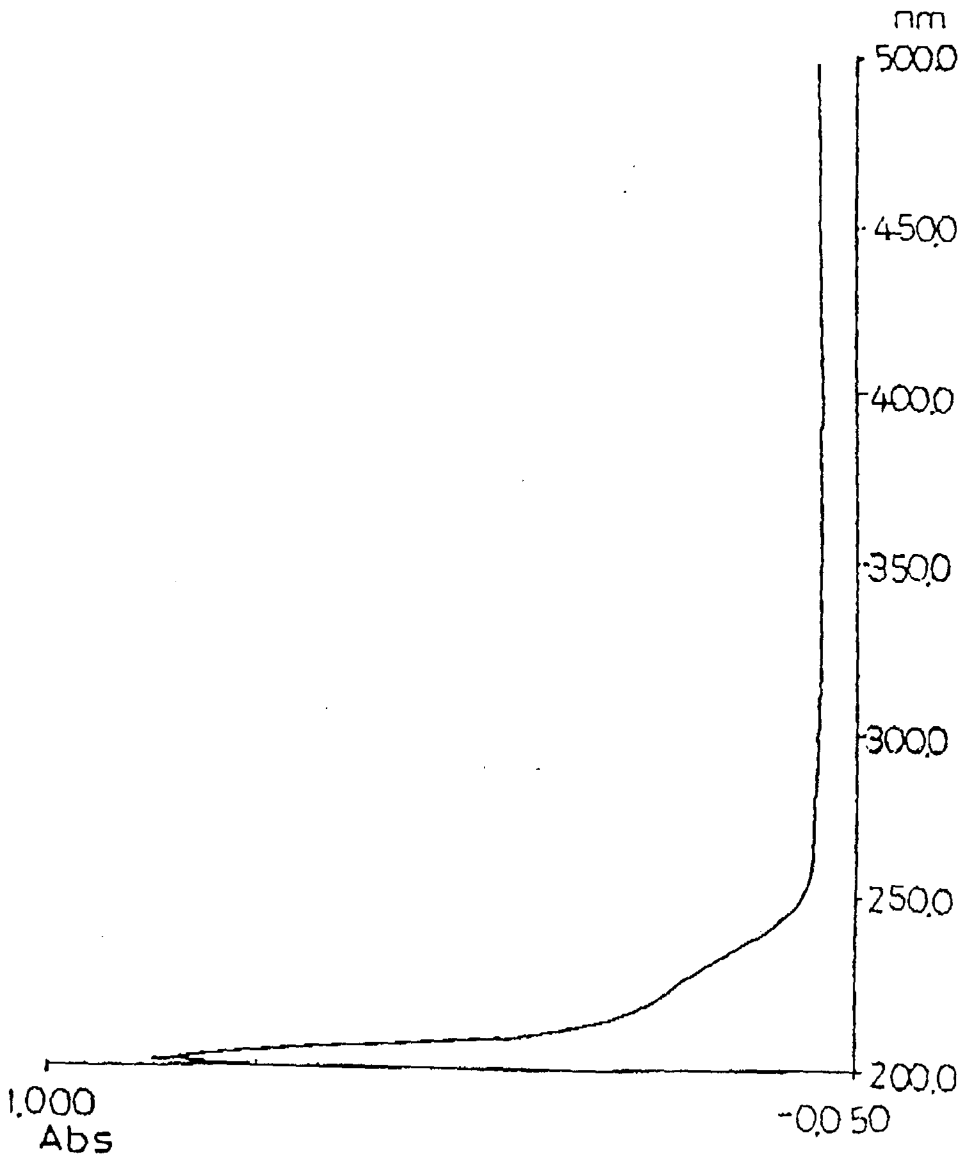


Fig. 1

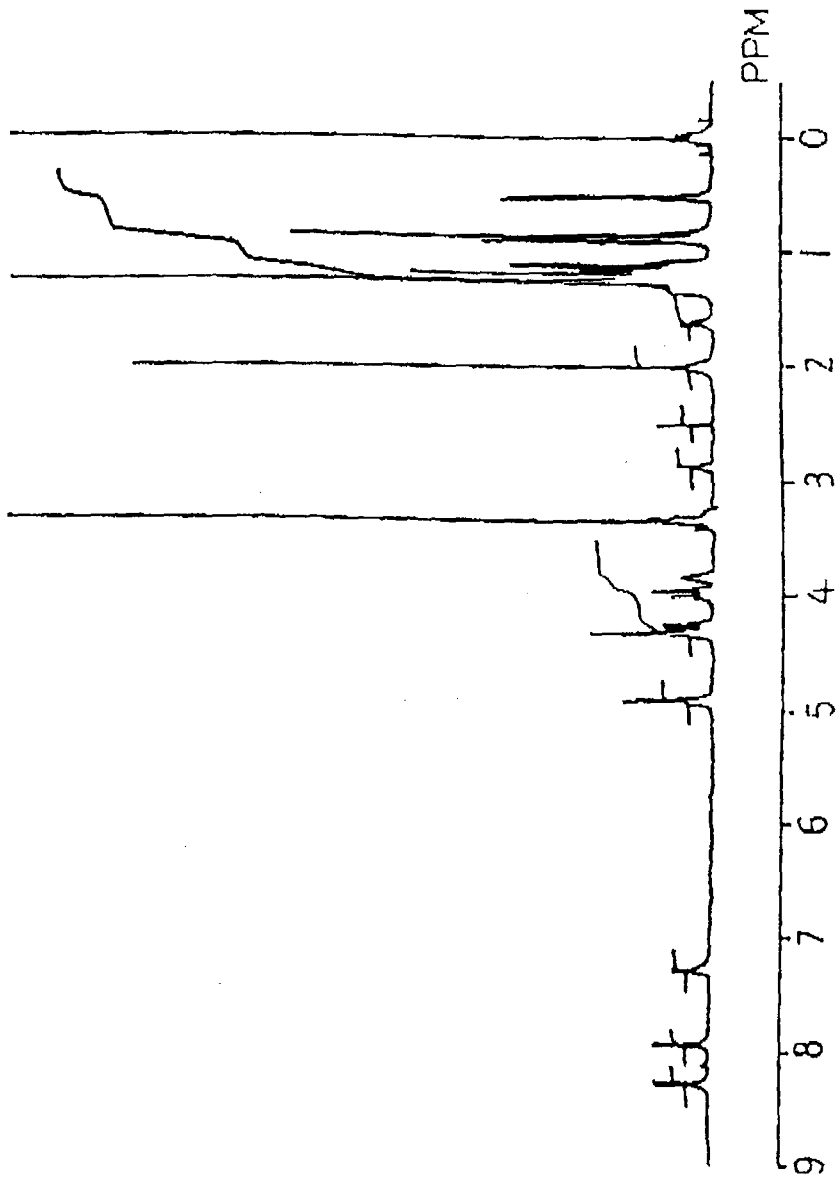


Fig. 2

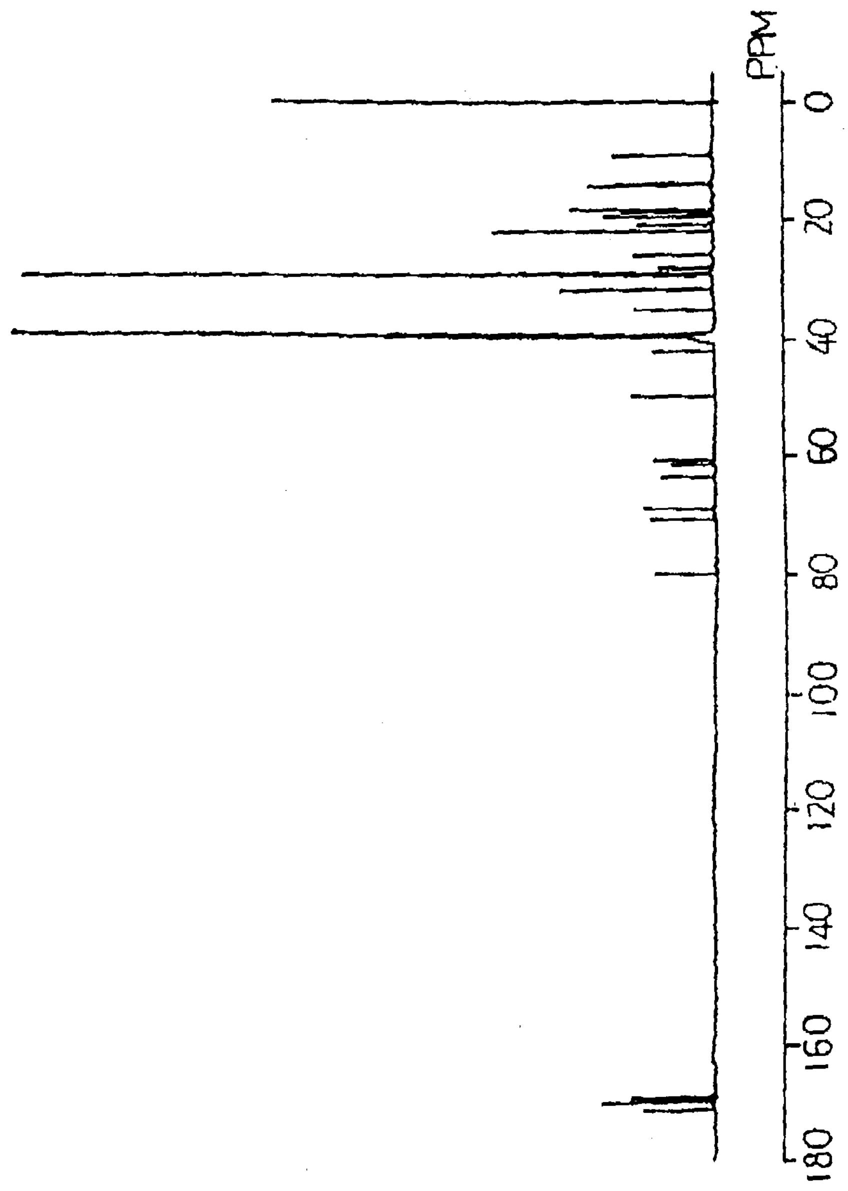


Fig. 3

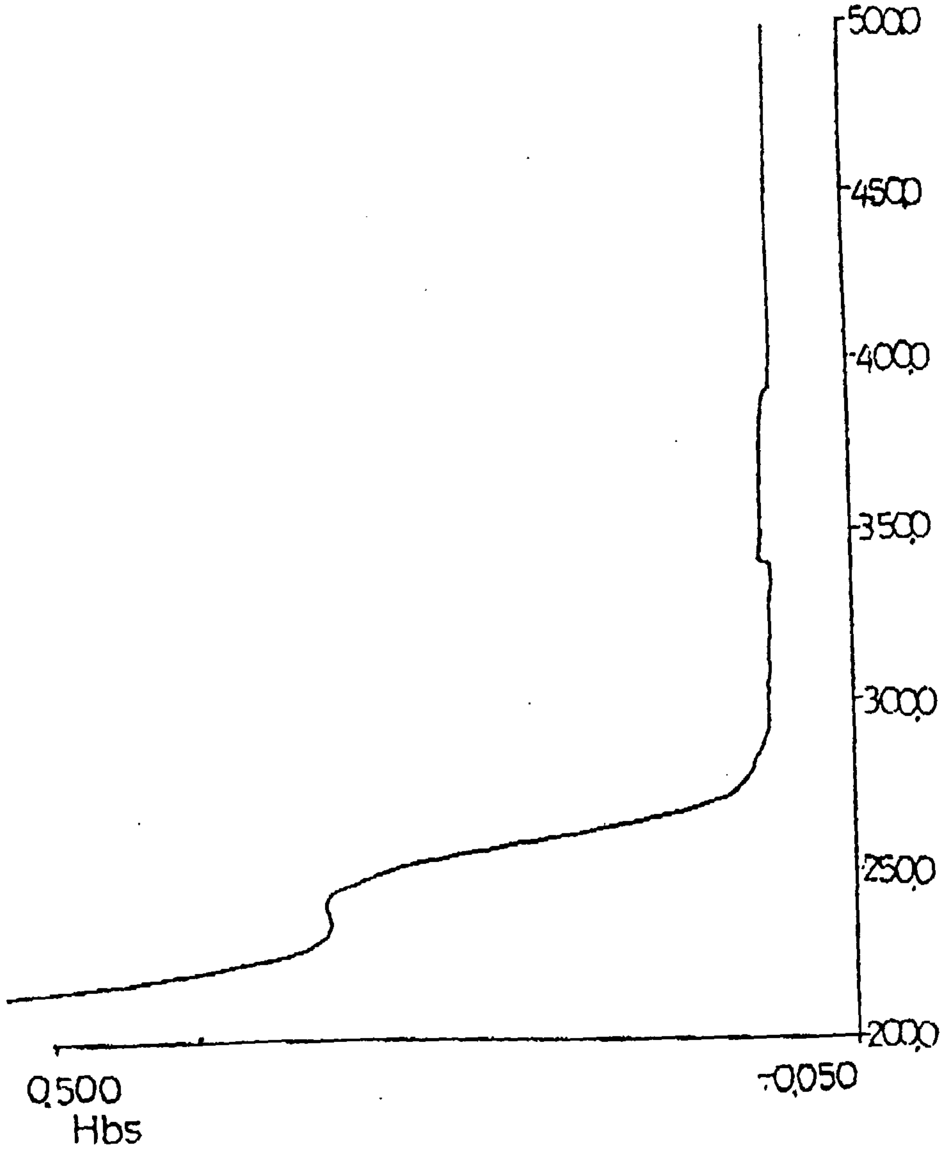


Fig. 4

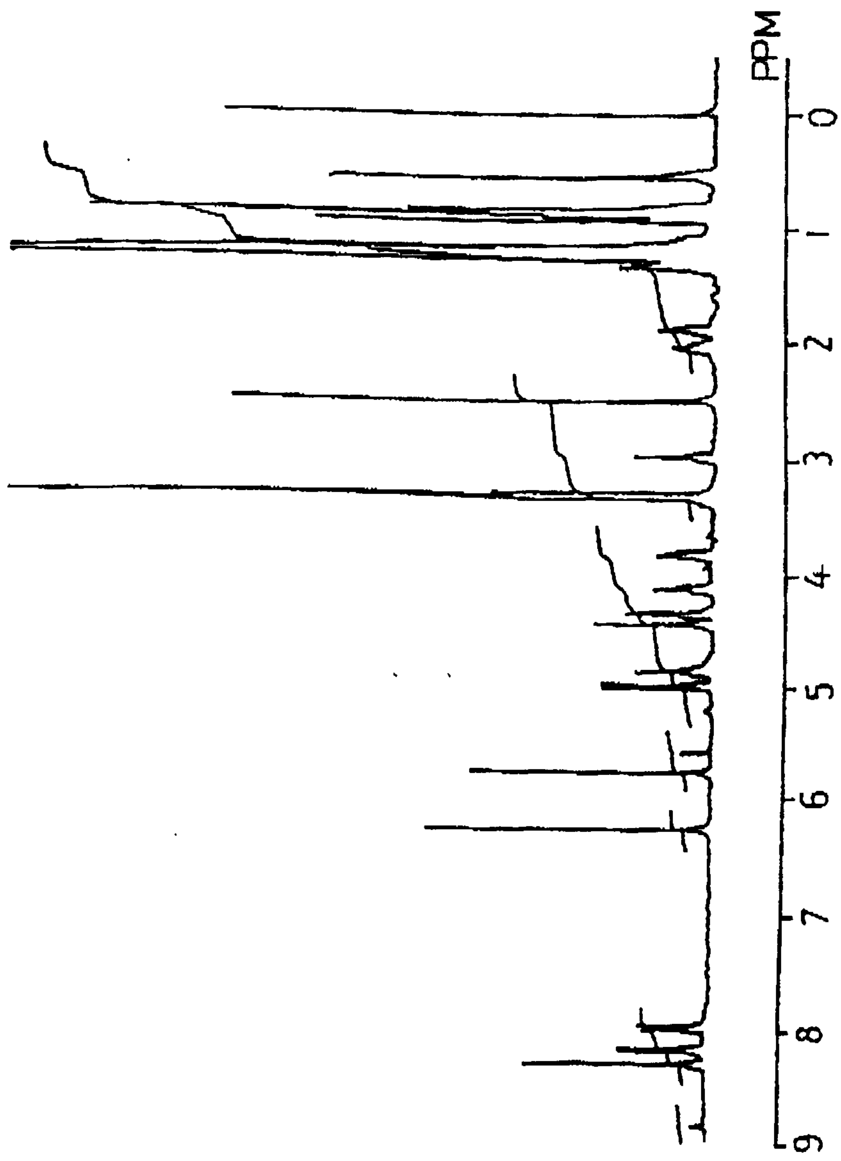


F i g. 5

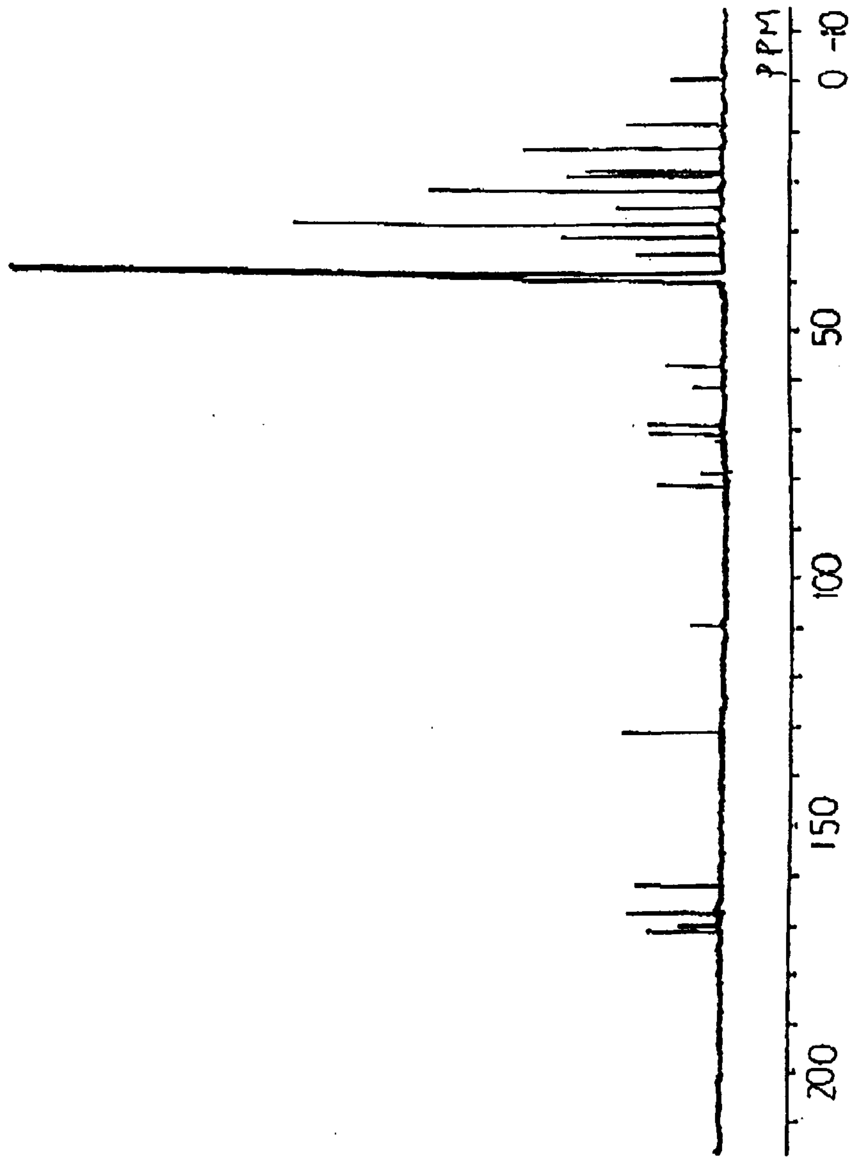


Fig. 6

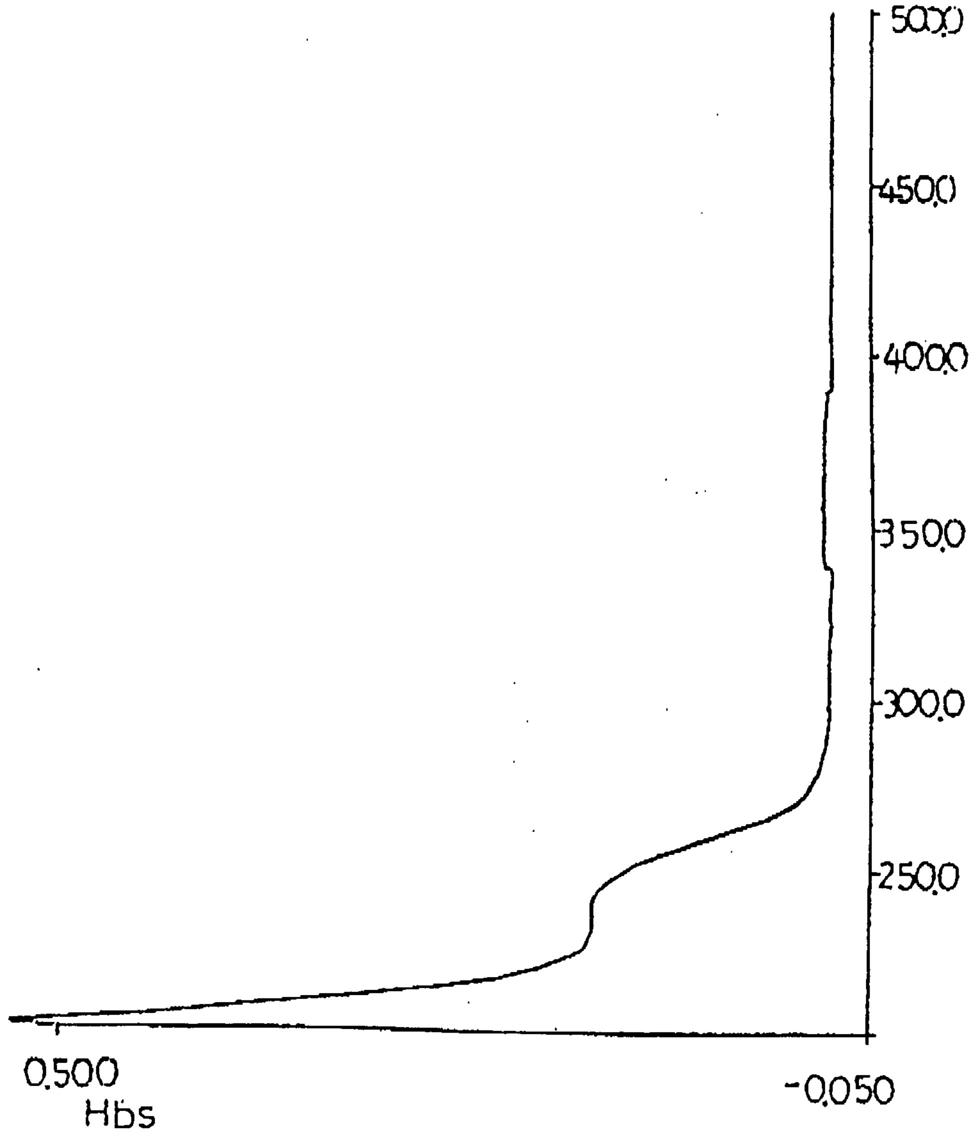


Fig. 7

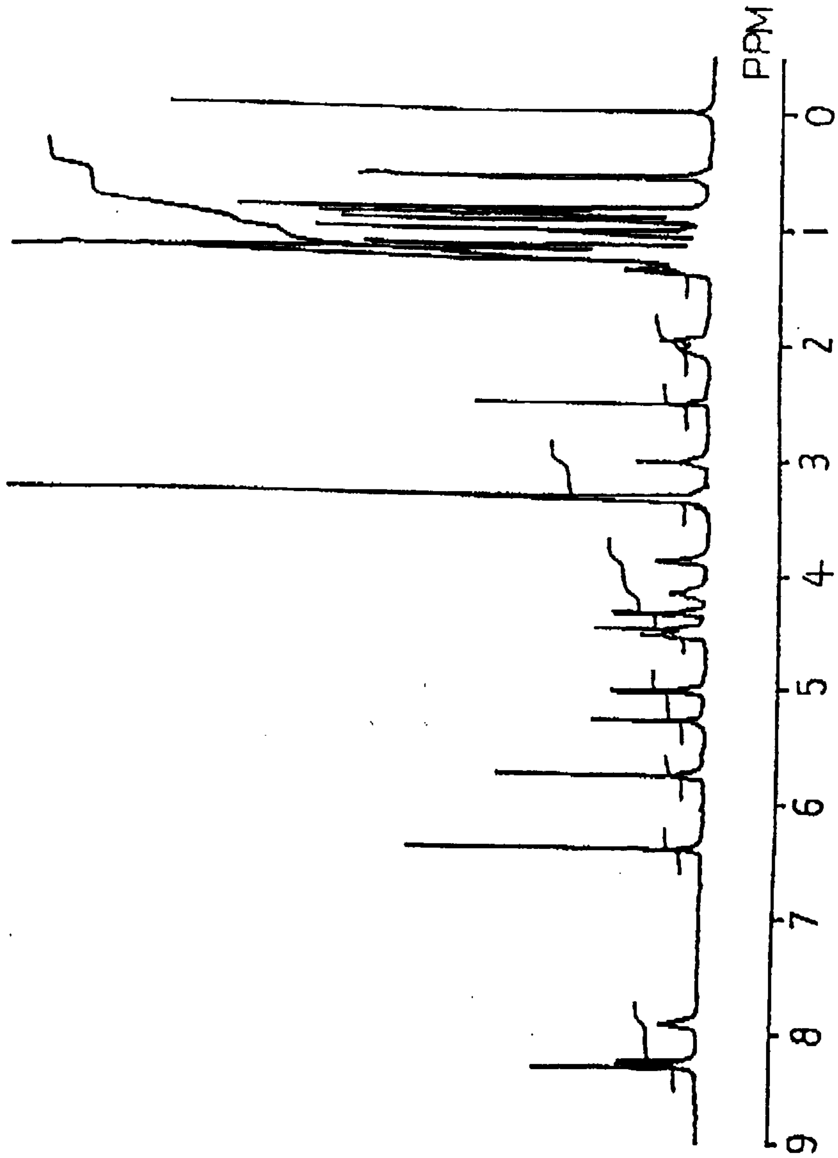


Fig. 8

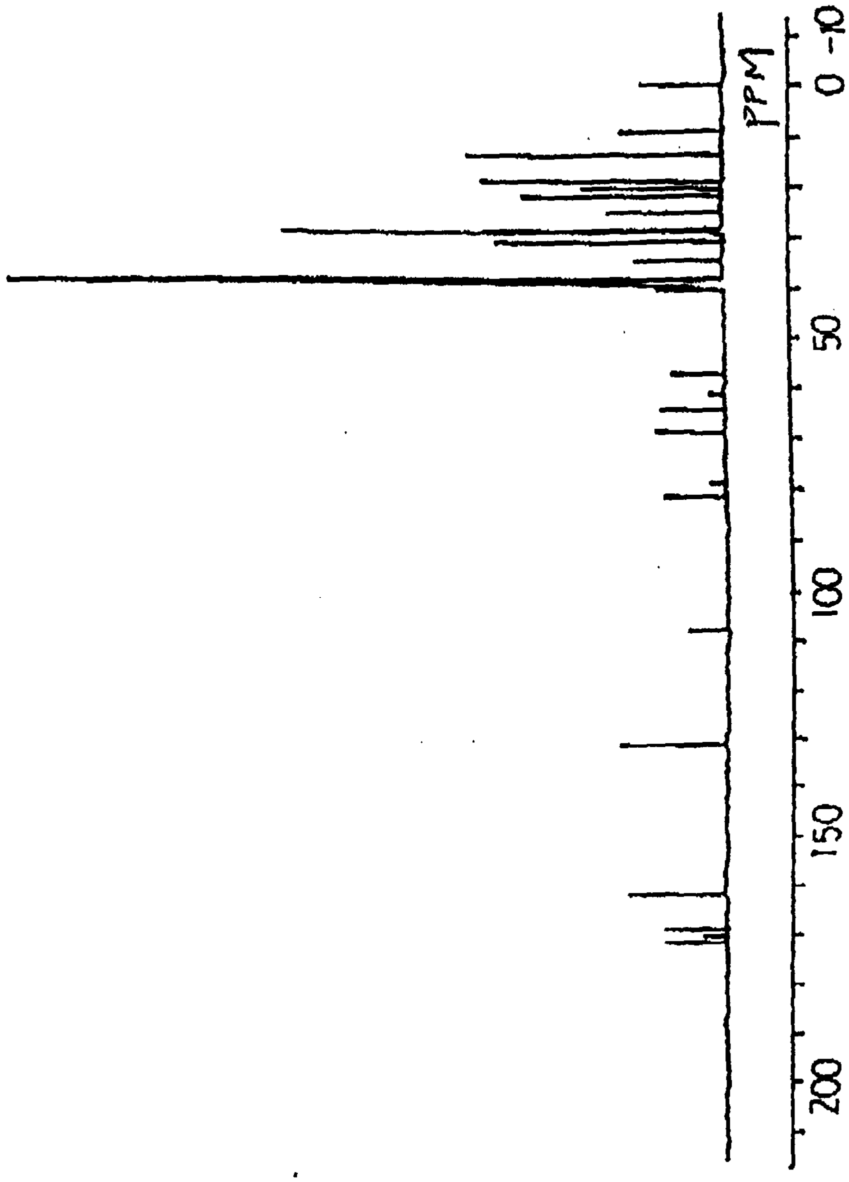


Fig. 9

## INTERNATIONAL SEARCH REPORT

International application No.
PCT/JP96/00275

**A. CLASSIFICATION OF SUBJECT MATTER**

Int. Cl$^6$ C07K5/12, C12P21/04, A61K38/12// (C12P21/04, C12R1:80)

According to International Patent Classification (IPC) or to both national classification and IPC

**B. FIELDS SEARCHED**

Minimum documentation searched (classification system followed by classification symbols)

Int. Cl$^6$ C07K5/12, C12P21/04, A61K38/12

Documentation searched other than minimum documentation to the extent that such documents are included in the fields searched

Electronic data base consulted during the international search (name of data base and, where practicable, search terms used)

CAS ONLINE, WPI/L, WPI, BIOSIS PREVIEWS

**C. DOCUMENTS CONSIDERED TO BE RELEVANT**

| Category* | Citation of document, with indication, where appropriate, of the relevant passages | Relevant to claim No. |
|---|---|---|
| A/Y | JP, H05-194571, A (Nippon Kayaku Co., Ltd.), August 3, 1993 (03. 08. 39) & EP, 525361, A1 & US, 5306496, A | 2, 3/ 1, 4-7 |
| Y | JP, S53-47081, B (Prodotti Antibiotici S.p.A.), December 19, 1978 (19. 12. 78) & US, 3961047, A & DE, 2406628, A | 1, 4-7 |
| Y | JP, S50-17996, B (Fujisawa Pharmaceutical Co., Ltd.), June 25, 1975 (25. 06. 75) & US, 3923790, A & DE, 2150593, A | 1, 4-7 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| May 1, 1996 (01. 05. 96) | May 21, 1996 (21. 05. 96) |
| Name and mailing address of the ISA/ Japanese Patent Office | Authorized officer |
| Facsimile No. | Telephone No. |

Form PCT/ISA/210 (second sheet) (July 1992)